# FASCICULE DE BREVET EUROPEEN

(11) **EP 1 439 790 B1**
(45) Date de publication et mention de la délivrance du brevet: **23.11.2005**
(21) Numéro de dépôt: 02796821.3
(22) Date de dépôt: 23.10.2002
(51) Int. Cl.: A61B 17/70

(54) **APPAREIL DE MAINTIEN DU RACHIS A ASSEMBLAGE PAR COINCEMENT**
WIRBELSÄULENSTÜTZVORRICHTUNG, DIE MIT KLEMMEN ZUSAMMENGESETZT IST
VERTEBRAL COLUMN SUPPORT DEVICE WHICH IS ASSEMBLED BY MEANS OF CLAMPING

(30) Priorité: 30.10.2001 FR 0114289
(43) Date de publication de la demande: 28.07.2004
(73) Titulaire: Vitatech, F-74970 Marignier (FR)
(72) Inventeur: GRADEL, Thomas, F-74130 Ayze (FR); COTTIN, Philippe, F-78470 Saint Remy les Chevreuse (FR); JABY, Yves, F-75015 Paris (FR); LEMAIRE, Jean-Philippe, F-21910 Saulon la Chapelle (FR)
(74) Mandataire: Poncet, Jean-François
(86) Numéro de dépôt international: PCT/FR2002/003623
(87) Numéro de publication internationale: WO 2003/037198

(56) Documents cités:
- WO-A-00/15125
- WO-A-91/11967
- FR-A- 2 720 923

## Description

La présente invention a pour objet un appareil permettant d'assurer le maintien des vertèbres dans une position désirée. Un tel appareil est utilisé pour le traitement d'un rachis présentant une déviation anormale, d'origine dégénérative ou traumatique.

On peut par exemple traiter des arthroses ou des fractures vertébrales, corriger des déviations de colonne vertébrale telles que la scoliose, la lordose et la cyphose.

On connaît notamment du document US-A-4 648 388 un appareil de traitement du rachis comprenant des éléments d'ancrage dans les vertèbres, une tige de solidarisation à section circulaire et à surface extérieure lisse, et des coulisseaux de liaison pour relier des éléments d'ancrage à la tige de solidarisation. Les éléments d'ancrage sont des vis comportant trois parties principales, une première partie d'extrémité à filet hélicoïdal adapté pour une pénétration et une tenue dans l'os, une partie intermédiaire cylindrique lisse de diamètre réduit, et une seconde partie d'extrémité à filet hélicoïdal adapté pour le vissage d'un écrou de serrage. Les coulisseaux de liaison comportent une partie de serrage conformée pour entourer un tronçon de la tige de solidarisation, et une partie de liaison dépassant latéralement et percée de deux trous correspondants destinés à être traversés par la vis d'ancrage. On visse tout d'abord la vis d'ancrage dans l'os, on adapte ensuite le coulisseau de liaison sur la partie intermédiaire cylindrique de la vis d'ancrage, et on visse enfin l'écrou de serrage sur la seconde partie filetée de la vis de serrage pour plaquer le coulisseau de liaison contre une vertèbre et pour serrer simultanément le coulisseau de liaison autour de la tige de solidarisation.

Un tel appareil est destiné à assurer le maintien du rachis selon une courbure appropriée. Il s'avère toutefois que le maintien mécanique assuré par cet appareil n'est pas suffisant. En particulier, l'appui du coulisseau directement sur une vertèbre exclut toute possibilité de serrage efficace, par suite de la faible résistance mécanique à la compression de la vertèbre, de sorte qu'il existe un risque majeur de glissement et de rotation du coulisseau sur la tige de solidarisation. Egalement, cet appareil n'est pas adapté à la réduction tridimensionnelle de la partie supérieure du rachis, partie dans laquelle l'implantation de vis est exclue. En outre, l'effort de serrage de l'écrou augmente sensiblement la contrainte de traction exercée sur la vis, en s'ajoutant aux efforts de tenue de la tige de solidarisation, ce qui réduit d'autant la résistance mécanique de l'ancrage et favorise la nécrose de l'os autour de la vis. Et lorsque le coulisseau est en position sur la vis, il n'est plus possible d'engager latéralement la tige de solidarisation dans le coulisseau.

Le document EP-A-0 408 489 décrit un appareil permettant de relier deux vertèbres, au moyen de deux vis pédiculaires à double filetage et de deux coulisseaux reliés par une tige filetée permettant d'en régler l'écartement. La vis comporte un plateau intermédiaire d'arrêt à portée sphérique mâle permettant un réglage d'inclinaison par rapport au coulisseau en s'engageant partiellement dans un trou du coulisseau. Le serrage d'un écrou de serrage sur la partie filetée de vis assure un blocage en rotation, l'écrou venant en appui sur une rondelle interposée contre le coulisseau. Ce dispositif, qui semble adapté pour le réglage d'espacement de deux vertèbres successives, est totalement inadapté pour tenir plus de deux vertèbres, et ne comporte aucun enseignement relatif au problème de l'insuffisance de tenue mécanique de tiges de solidarisation lisses. Et lorsque le coulisseau est en position sur la vis, il n'est plus possible d'engager latéralement la tige filetée.

Le document WO 91 11967 (Le preambule de la revendication 1 est basé sur ce document.) décrit un appareil de traitement du rachis comprenant des vis pédiculaires à double filetage et plateau intermédiaire d'arrêt, sur lesquelles on rapporte un coulisseau conformé pour recevoir et retenir une tige de solidarisation. Le coulisseau comporte une rainure inférieure dans laquelle s'engage le plateau d'arrêt pour éviter sa rotation par rapport à la vis, et comporte une rainure supérieure dans laquelle s'engage la tige de solidarisation. La partie filetée de vis traverse le coulisseau, et un écrou de blocage à portée inférieure tronconique se visse sur la partie filetée et force latéralement la tige de solidarisation dans la rainure supérieure du coulisseau pour son blocage. La portée conique de l'écrou, en appui sur la forme cylindrique de tige de solidarisation, ne permet pas d'assurer une solidarisation suffisamment rigide de la tige de solidarisation. En outre, lorsque le coulisseau est fixé sur la vis par l'écrou, il n'est plus possible d'engager latéralement la tige de solidarisation.

Le document WO 00 15125 décrit deux autres moyens de solidarisation de la tige sur le coulisseau. Le premier moyen est une vis pointeau à extrémité conique portant sur le côté de la tige. Le second moyen est un cavalier poussé par une vis radialement contre le côté de la tige. Aucun de ces moyens n'assure une solidarisation suffisamment rigide.

Dans le document FR 2 720 923, un écrou de blocage à portée sphérique vient en appui radial contre la tige. Pour les mêmes raisons que précédemment, la solidarisation n'est pas suffisamment rigide.

### EXPOSE DE L'INVENTION

Le problème proposé par la présente invention est de concevoir une nouvelle structure d'appareil de traitement de rachis à tige de solidarisation lisse et coulisseaux de liaison pour éléments d'ancrage, qui assure une solidarisation nettement plus efficace des vertèbres et une plus grande facilité de pose et de réglage de position des éléments les uns par rapport aux autres en tridimensionnel. On cherche en particulier à autoriser l'amenée et le retrait de la tige de solidarisation par déplacement latéral sur un coulisseau déjà en position sur une vis ou autre élément d'ancrage.

Pour cela, l'appareil de traitement du rachis selon la présente invention comprend :
- des éléments d'ancrage dans les vertèbres, comportant chacun une partie cylindrique filetée sur laquelle se visse un écrou de serrage, et comportant une partie d'ancrage reliée à la partie cylindrique filetée par une collerette d'arrêt intermédiaire,
- au moins une tige de solidarisation, à section circulaire et à surface extérieure lisse,
- des coulisseaux de liaison pour relier des éléments d'ancrage à la tige de solidarisation, les coulisseaux de liaison comportant un premier trou conformé pour le passage de la partie cylindrique filetée d'élément d'ancrage pour assurer leur solidarisation à un élément d'ancrage, les coulisseaux de liaison comportant une rainure transversale supérieure conformée pour recevoir un tronçon de la tige de solidarisation, avec des moyens de serrage pour sélectivement serrer ou desserrer la tige de solidarisation dans ladite rainure transversale ;

selon l'invention les moyens de serrage comprennent :
- une vis de serrage, comportant une tige filetée et une tête,
- un trou de serrage distinct du premier trou, et prévu dans le fond de rainure transversale du coulisseau et déporté latéralement par rapport au premier trou,
- une portée cylindrique constituant un premier bord de la rainure transversale et conformée pour recevoir le tronçon de tige de solidarisation,
- une portée oblique constituant un bord opposé de la rainure transversale et inclinée par rapport à l'axe du trou de serrage,
- un cavalier engagé en coin dans la rainure transversale entre la portée oblique et la tige de solidarisation et repoussé vers le fond de la rainure transversale par la vis de serrage, avec une face d'appui en appui glissant sur la portée oblique, et avec une face de poussée opposée en appui sur la tige de solidarisation pour assurer son blocage dans la rainure transversale.

Selon une réalisation pratique, le cavalier est percé d'un trou traversé par la vis de serrage, de sorte que la tête de la vis de serrage est en appui sur la face externe du cavalier pour le repousser vers le fond de la rainure transversale.

De préférence, le trou de serrage est orienté obliquement par rapport à l'axe du premier trou, dans le sens du rapprochement vers l'axe du premier trou lors du vissage de la vis de serrage.

Pour améliorer à la fois le maintien rigide de la tige de solidarisation et la facilité d'introduction latérale de la tige de solidarisation dans le coulisseau, la face de poussée comprend une partie inférieure plane et orientée généralement vers le fond de la rainure transversale pour être en appui contre la tige de solidarisation, et une partie supérieure ouverte vers le haut pour faciliter l'engagement latéral de la tige de solidarisation.

Selon un mode de réalisation avantageux, facilitant les opérations d'assemblage des éléments de l'appareil après fixation des éléments d'ancrage sur le rachis, on prévoit des moyens permettant d'orienter le coulisseau sur l'élément d'ancrage correspondant. Pour cela, la collerette d'arrêt comporte une surface d'appui sphérique mâle venant s'engager dans une surface sphérique femelle correspondante à l'entrée du premier trou du coulisseau de liaison, le premier trou du coulisseau de liaison ayant par ailleurs un diamètre supérieur au diamètre de partie cylindrique filetée d'élément d'ancrage pour autoriser une oscillation angulaire du coulisseau de liaison à l'écart de l'axe sur l'élément d'ancrage avant serrage de l'écrou de serrage.

L'appareil peut comprendre, comme élément d'ancrage, une ou plusieurs vis osseuses à double filetage de part et d'autre d'une collerette d'arrêt intermédiaire, un ou plusieurs crochets dont la paroi arrière sert de collerette d'arrêt, et éventuellement des plaques d'appui conformées pour s'adapter à la morphologie particulière de certaines zones du rachis.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles:
- la figure 1 est une vue en perspective d'une vis osseuse pouvant servir d'élément d'ancrage selon l'invention, assemblée à un coulisseau et à une tige de solidarisation selon un mode de réalisation préféré ;
- la figure 2 est une vue en coupe transversale à plus grande échelle de l'ensemble de la figure 1 ; et
- la figure 3 est une vue en perspective d'un crochet lamaire pouvant servir d'élément d'ancrage selon l'invention, assemblé à un coulisseau pour adaptation sur une tige de solidarisation.

### DESCRIPTION DES MODES DE REALISATION PREFERES

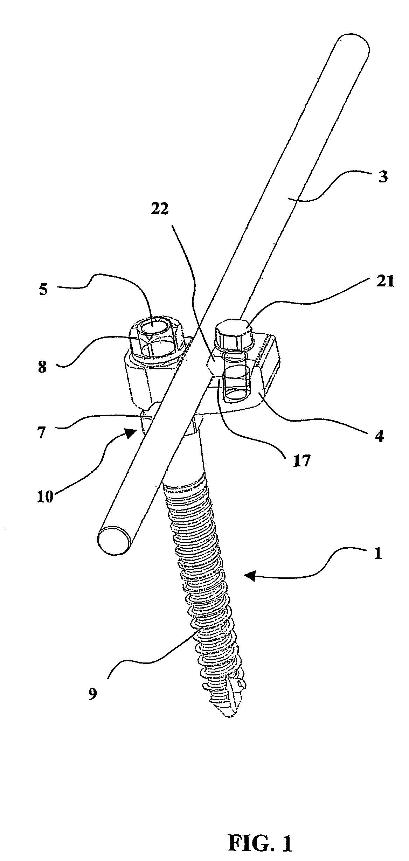

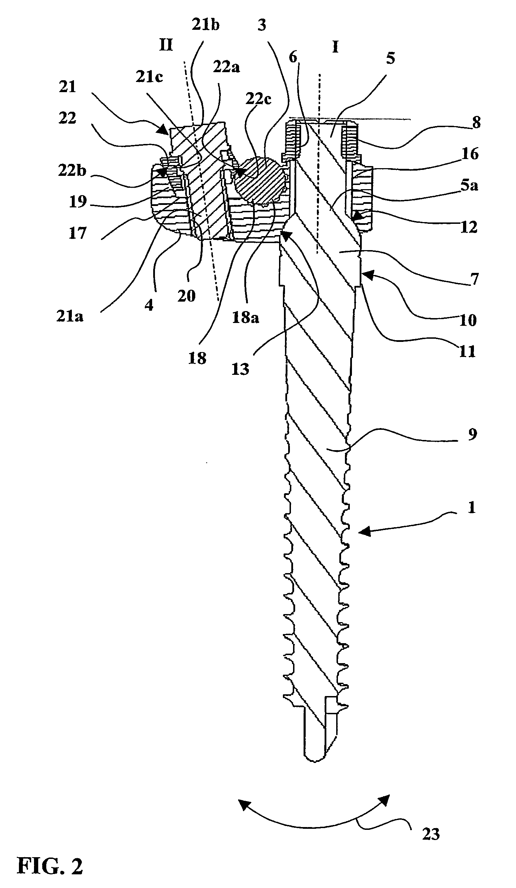

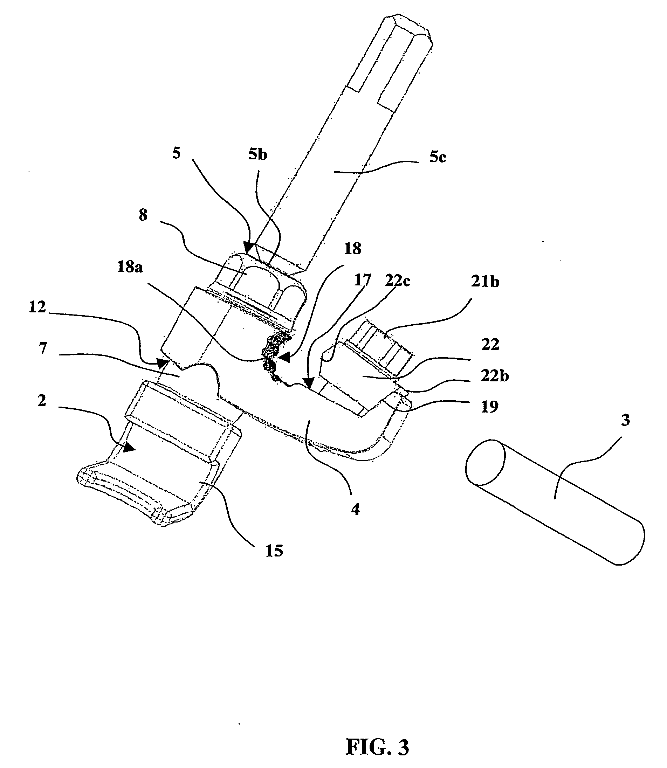

L'appareil représenté sur les figures comprend des éléments d'ancrage tels que des vis osseuses 1 (figures 1 et 2) ou des crochets 2 (figure 3), des tiges de solidarisation 3, et des coulisseaux de liaison 4 entre chaque élément d'ancrage et la tige de solidarisation 3.

Les tiges de solidarisation 3 de l'appareil selon l'invention sont des tiges lisses, de section circulaire. On choisit le matériau et la section de manière à obtenir une élasticité optimale, adaptée aux efforts à supporter lors de l'utilisation, et de façon que la tige puisse être galbée selon la forme souhaitée pour la partie de colonne vertébrale à traiter.

Chaque élément d'ancrage, vis osseuse 1, crochet 2 ou plaque d'appui, est d'une seule pièce avec une partie cylindrique filetée 5 pourvue d'un filetage extérieur 6, et comporte une collerette d'arrêt 7 limitant le serrage d'un écrou de serrage 8 sur ce filetage extérieur 6.

Dans le mode de réalisation des figures 1 et 2, l'élément d'ancrage est une vis osseuse 1 à double filetage, la partie cylindrique filetée 5 à filetage extérieur 6 constituant le premier filetage, une seconde partie coaxiale filetée 9, à l'opposé de la collerette d'arrêt 7, constituant un second filetage adapté pour se visser dans l'os. La collerette d'arrêt 7 constitue une partie intermédiaire de plus grande section séparant les deux parties filetées 5 et 9. La collerette d'arrêt 7 comprend avantageusement une surface périphérique 10 à facettes en six pans, une surface de butée 11 se raccordant à la seconde partie coaxiale filetée 9 et destinée à venir porter contre l'os de vertèbre, et une surface d'appui 12 opposée se raccordant à la partie cylindrique filetée 5 et se développant à partir de sa base 5a.

La surface d'appui 12 de la collerette d'arrêt 7 est, dans le mode de réalisation représenté, une surface convexe sphérique dont la convexité est tournée vers la partie cylindrique filetée 5, et qui constitue une surface d'appui sphérique mâle susceptible de venir s'engager dans une surface sphérique femelle correspondante 13 du coulisseau de liaison 4.

De façon connue, la partie cylindrique filetée 5 peut être prolongée par un tronçon fileté coaxial excédentaire 5c (représenté sur la figure 3) et auquel elle se raccorde par une gorge annulaire intermédiaire 5b constituant une amorce de rupture pour faciliter le sectionnement du tronçon excédentaire après vissage d'un écrou sur la partie cylindrique filetée 5. La gorge annulaire 5b présente avantageusement une section en dent de scie, le fond de gorge étant adjacent à l'extrémité de partie cylindrique filetée 5.

Dans le mode de réalisation de la figure 3, l'élément d'ancrage est un crochet 2 pédiculaire, destiné à accrocher le pédicule d'une vertèbre. Le crochet 2 comporte un corps de crochet 15, recourbé comme le représente la figure, et une surface d'appui 12 sur laquelle se raccorde la partie cylindrique filetée 5. Comme dans le cas de la vis pédiculaire de la figure 2, la surface d'appui 12 est une surface sphérique mâle.

Le coulisseau de liaison 4 comporte un premier trou 16 conformé pour le passage de la partie cylindrique filetée 5 d'un élément d'ancrage 1 ou 2, et comporte une rainure transversale 17 supérieure conformée pour recevoir un tronçon de la tige de solidarisation 3. Des moyens de serrage permettent de serrer ou de desserrer sélectivement la tige de solidarisation 3 dans la rainure transversale 17.

La rainure transversale 17 comprend une portée cylindrique 18 qui constitue l'un de ses bords et qui est conformée pour recevoir le tronçon de tige de solidarisation 3, par exemple en l'enveloppant sur environ 120° comme illustré sur la figure 2. De préférence, la portée cylindrique 18 comporte des reliefs antiglissement 18a qui s'opposent efficacement à tout déplacement de la tige de solidarisation 3 dans le coulisseau de liaison 4 tant en translation qu'en rotation.

Le bord opposé de la rainure transversale 17 est constitué par une portée oblique 19, inclinée de façon que la rainure transversale 17 soit évasée vers le haut.

Le coulisseau de liaison 4 comporte un trou de serrage 20 taraudé, distinct du premier trou 16, et prévu dans le fond de la rainure transversale 17 du coulisseau de liaison 4.

Une vis de serrage 21, comportant une tige filetée 21a et une tête 21b, se visse dans le trou de serrage 20.

Le trou de serrage 20 est déporté latéralement par rapport au premier trou 16, et son axe II est de préférence légèrement oblique par rapport à l'axe I du premier trou 16, les axes I et II formant un angle dont le sommet est dirigé vers le bas c'est-à-dire à l'opposé de la rainure transversale 17.

Ainsi le trou de serrage 20 est orienté obliquement par rapport à l'axe I du premier trou 16 dans le sens du rapprochement vers l'axe I du premier trou 16 lors du vissage de la vis de serrage 21.

La portée oblique 19 qui limite le bord opposé de la rainure transversale 17 est inclinée de façon à former, avec l'axe II du trou de serrage 20, un angle dont le sommet est également dirigé vers le bas.

Un cavalier 22 est engagé en coin dans la rainure transversale 17 entre la portée oblique 19 et la tige de solidarisation 3. Le cavalier 22 est repoussé vers le fond de la rainure transversale 17 par la vis de serrage 21. Pour cela, le cavalier 22 est percé d'un trou traversé par la vis de serrage 21, de sorte que la tête 21b de la vis de serrage 21 est en appui sur la face externe du cavalier 22 pour le repousser vers le fond de la rainure transversale 17.

De préférence, le cavalier 22 tourne librement autour de la vis de serrage 21, et est retenu axialement sur la tige filetée 21a de la vis de serrage 21 par une collerette 21c de la vis de serrage 21 qui est engagée dans une rainure annulaire intérieure 22a ovale du trou du cavalier 22.

Le cavalier 22 comprend une face d'appui 22b qui est en appui glissant sur la portée oblique 19 du coulisseau de liaison 4, et comprend une face de poussée 22c opposée en appui sur la tige de solidarisation 3 pour assurer son blocage dans la rainure transversale 17. La face de poussée 22c comprend avantageusement une partie inférieure plane et orientée généralement vers le fond de la rainure transversale 17 pour être en appui contre une partie supérieure de la tige de solidarisation 3, et une partie supérieure ouverte vers le haut pour faciliter l'engagement latéral de la tige de solidarisation 3.

L'extrémité inférieure du premier trou 16 est évasée pour constituer la surface sphérique femelle 13 dans laquelle vient s'engager la surface d'appui 12 sphérique mâle de la collerette d'arrêt 7. Le premier trou 16 du coulisseau de liaison 4 a par ailleurs un diamètre supérieur au diamètre de partie cylindrique filetée 5 d'élément d'ancrage 1 ou 2, de façon à autoriser une oscillation angulaire du coulisseau de liaison 4 à l'écart de l'axe I sur l'élément d'ancrage 1 ou 2 avant serrage de l'écrou de serrage 8. L'oscillation est illustrée schématiquement par la double flèche 23 sur la figure 2.

Dans le mode de réalisation illustré sur les figures, dans la rainure transversale 17 la tige de solidarisation 3 est engagée en position intermédiaire entre la vis de serrage 21 et la partie munie du premier trou 16 pour recevoir la partie cylindrique filetée 5 de l'élément d'ancrage 1 ou 2.

L'appareil de l'invention permet l'utilisation d'une tige ou de plusieurs éléments de tige, de courbure appropriée à la région vertébrale à traiter.

La mise en place des coulisseaux de liaison 4 sur la tige de solidarisation 3 s'effectue aisément, puisqu'ils peuvent être enfilés par chacune des extrémités de cette dernière, ou adaptés par engagement latéral, et ils coulissent librement sur la tige de solidarisation 3 quelle que soit sa courbure.

Le principe de réduction du rachis déformé est tridimensionnel. Il faut transformer une courbe scoliotique orientée dans un plan proche du plan frontal en une courbe d'allure physiologique située dans le plan sagittal et présentant une courbure cyphotique, thoracique et lordotique lombaire normale.

On donne tout d'abord à la tige de solidarisation une forme proche de la courbure physiologique normale, et on la positionne dans le plan sagittal du patient, en fixant ses deux extrémités par des éléments d'ancrage et des coulisseaux correctement serrés. Les reliefs antiglissement des coulisseaux permettent ainsi un blocage très efficace de la tige, à la fois en translation et surtout en rotation. On positionne des éléments d'ancrage dans ou sur les autres vertèbres intermédiaires. Les ancrages les plus proches de la tige sont engagés dans les coulisseaux et verrouillés en bloquant également la tige dans le plan sagittal et en rotation par serrage de la vis de serrage. L'engagement des ancrages les plus éloignés situés dans la concavité de la courbe est permis par la longueur de la partie cylindrique filetée 5 des éléments d'ancrage. Une fois l'extrémité de la partie cylindrique filetée passée dans l'alésage du coulisseau de liaison 4 lui-même fixé sur la tige, le serrage progressif de l'écrou de serrage 8 rapproche le point d'ancrage de la tige de solidarisation, réalisant la réduction progressive de courbure vertébrale. Si l'élasticité du rachis s'épuise, la tige de solidarisation 3 se déforme, et assure une correction dans une position intermédiaire de réduction maximale.

L'appareil selon l'invention permet ainsi d'aller rechercher sur place chaque élément d'ancrage intermédiaire, pour introduire sa partie cylindrique filetée dans l'alésage d'un coulisseau, et de le fixer rigidement sur la tige, en alignement avec les éléments d'ancrage supérieur et inférieur, pour obtenir le redressement voulu des vertèbres ainsi appareillées.

La mise en place de l'appareil faisant l'objet de l'invention sur le rachis du patient peut s'effectuer aussi bien en position antérieure qu'en position postérieure, par rapport au rachis.

La mise en place de l'appareil selon l'invention peut s'effectuer dans un temps opératoire nettement plus court qu'avec les appareils connus antérieurement, étant donné la facilité accrue avec laquelle le chirurgien peut présenter et assembler les éléments de l'appareil les uns aux autres et au rachis.

On comprendra qu'un appareil selon l'invention peut comprendre, selon le traitement de rachis à réaliser, des ancrages à vis, ou des ancrages à crochets, ou des ancrages à plaques, ou une combinaison de deux ou trois de ces types d'ancrage qui sont ainsi indépendants les uns des autres.

On peut notamment prévoir, comme élément d'ancrage, une ou plusieurs vis osseuses à double filetage, la partie cylindrique filetée d'élément d'ancrage constituant le premier filetage, une seconde partie coaxiale filetée, à l'opposé de la collerette d'arrêt, constituant un second filetage adapté pour se visser dans l'os.

En alternative ou en complément, on peut prévoir, comme élément d'ancrage, un ou plusieurs crochets dans lesquels la paroi arrière des crochets, perpendiculaire à l'axe de la partie cylindrique filetée, sert de collerette d'arrêt.

La présente invention n'est pas limitée aux modes de réalisation qui ont été explicitement décrits, mais elle en inclut les diverses variantes et généralisations contenues dans le domaine des revendications ci-après.

## Revendications

1. Appareil de traitement du rachis, comprenant :
- des éléments d'ancrage (1, 2) dans les vertèbres, comportant chacun une partie cylindrique filetée (5) sur laquelle se visse un écrou de serrage (8), et comportant une partie d'ancrage reliée à la partie cylindrique filetée (5) par une collerette d'arrêt (7) intermédiaire,
- au moins une tige de solidarisation (3), à section circulaire et à surface extérieure lisse,
- des coulisseaux de liaison (4) pour relier des éléments d'ancrage (1, 2) à la tige de solidarisation (3), les coulisseaux de liaison (4) comportant un premier trou (16) conformé pour le passage de la partie cylindrique filetée (5) d'élément d'ancrage pour assurer leur solidarisation à un élément d'ancrage, les coulisseaux de liaison comportant une rainure transversale (17) supérieure conformée pour recevoir un tronçon de la tige de solidarisation (3), avec des moyens de serrage pour sélectivement serrer ou desserrer la tige de solidarisation (3) dans ladite rainure transversale (17),
**caractérisé en ce que** les moyens de serrage comprennent :
- une vis de serrage (21), comportant une tige filetée et une tête,
- un trou de serrage (20) distinct du premier trou (16), prévu dans le fond de rainure transversale du coulisseau et déporté latéralement par rapport au premier trou (16),
- une portée cylindrique (18) constituant un premier bord de la rainure transversale (17) et conformée pour recevoir le tronçon de tige de solidarisation (3),
- une portée oblique (19) constituant un bord opposé de la rainure transversale (17) et inclinée par rapport à l'axe du trou de serrage,
- un cavalier (22) engagé en coin dans la rainure transversale (17) entre la portée oblique (19) et la tige de solidarisation (3) et repoussé vers le fond de la rainure transversale (17) par la vis de serrage (21), avec une face d'appui (22b) en appui glissant sur la portée oblique (19), et avec une face de poussée (22c) opposée en appui sur la tige de solidarisation (3) pour assurer son blocage dans la rainure transversale (17).

2. Appareil de traitement du rachis selon la revendication 1, **caractérisé en ce que** le cavalier (22) est percé d'un trou traversé par la vis de serrage (21), de sorte que la tête (21b) de la vis de serrage (21) est en appui sur la face externe du cavalier (22) pour le repousser vers le fond de la rainure transversale (17).

3. Appareil de traitement du rachis selon l'une des revendications 1 ou 2, **caractérisé en ce que** le trou de serrage (20) est orienté obliquement par rapport à l'axe (I) du premier trou (16), dans le sens du rapprochement vers l'axe (I) du premier trou (16) lors du vissage de la vis de serrage (21).

4. Appareil de traitement du rachis selon l'une quelconque des revendications 1 à 3, **caractérisé en ce que** la face de poussée (22c) comprend une partie inférieure plane et orientée généralement vers le fond de la rainure transversale (17) pour être en appui contre la tige de solidarisation (3), et une partie supérieure ouverte vers le haut pour faciliter l'engagement latéral de la tige de solidarisation (3).

5. Appareil de traitement du rachis selon l'une quelconque des revendications 1 à 4, **caractérisé en ce que** le cavalier (22) tourne librement autour de la vis de serrage (21), et est retenu axialement sur la tige filetée (21a) de la vis de serrage (21) par une collerette (21c) de la vis de serrage (21) engagée dans une rainure annulaire intérieure (22a) ovale du trou du cavalier (22).

6. Appareil de traitement du rachis selon l'une quelconque des revendications 1 à 5, **caractérisé en ce que** la collerette d'arrêt (7) comporte une surface d'appui (12) sphérique mâle venant s'engager dans une surface sphérique femelle (13) correspondante à l'entrée du premier trou (16) du coulisseau de liaison (4), le premier trou (16) du coulisseau de liaison (4) ayant par ailleurs un diamètre supérieur au diamètre de partie cylindrique filetée (5) d'élément d'ancrage pour autoriser une oscillation angulaire du coulisseau de liaison (4) à l'écart de l'axe (I) sur l'élément d'ancrage avant serrage de l'écrou de serrage (8).

## Patentansprüche

1. Vorrichtung zur Behandlung der Wirbelsäule, umfassend :
- Elemente (1, 2) zur Verankerung in den Wirbeln, von denen jedes einen zylindrischen Gewindeabschnitt (5), auf den eine Klemmmutter (8) aufgeschraubt ist, sowie einen Verankerungsteil aufweist, der mit dem zylindrischen Gewindeabschnitt (5) über einen dazwischenliegenden Endanschlagkragen (7) verbunden ist,
- wenigstens eine Stabilisierungsstange (3) mit kreisförmigem Querschnitt und glatter Außenfläche,
- Verbindungsschieber (4) zum Verbinden der Verankerungselemente (1, 2) mit der Stabilisierungsstange (3), wobei die Verbindungsschieber (4) eine erste Bohrung (16) für den Durchtritt des zylindrischen Gewindeabschnitts (5) eines Verankerungselements zur Herstellung ihrer festen Verbindung mit einem Verankerungselement sowie eine obere Querrinne (17) aufweisen, die zur Aufnahme eines Abschnitts der Stabilisierungsstange (3) über Klemmmittel ausgebildet ist, um durch diese die Stabilisierungsstange (3) in der Querrinne (17) wahlweise festzuklemmen oder zu lösen,
**dadurch gekennzeichnet, daß** die Klemmmittel aufweisen :
- eine Klemmschraube (21) mit einem Gewindeschaft und einem Kopf,
- eine von der ersten Bohrung (16) verschiedene Spannbohrung (20), die in den Boden der Querrinne des Verbindungsschiebers eingearbeitet und bezüglich der ersten Bohrung (16) seitlich versetzt ist,
- eine zylindrische Fläche (18), die einen ersten Rand der Querrinne (17) bildet und zur Aufnahme des Abschnitts der Stabilisierungsstange (3) ausgebildet ist,
- eine Schrägfläche (19), die einen gegenüberliegenden Rand der Querrinne (17) bildet und zur Achse der Spannbohrung geneigt ist,
- einen Einsatz (22), der in die Querrinne (17) zwischen die Schrägfläche (19) und die Stabilisierungsstange (3) mit Keilwirkung eingesetzt ist und in Richtung zum Boden der Querrinne (17) durch die Klemmschraube (21) gedrückt wird, wobei eine Stützfläche (22b) des Einsatzes reibschlüssig an der Schrägfläche (19) anliegt, während eine gegenüberliegende Druckfläche (22c) des Einsatzes an der Stabilisierungsstange (3) anliegt, wodurch diese in der Querrinne (17) blockiert wird.

2. Vorrichtung zur Behandlung der Wirbelsäule nach Anspruch 1, **dadurch gekennzeichnet, daß** in den Einsatz (22) eine Bohrung für den Durchtritt der Klemmschraube (21) eingearbeitet ist, derart, daß sich der Kopf (21b) der Klemmschraube (21) auf der Außenseite des Einsatzes (22) abstützt, um diesen in Richtung zum Boden der Querrinne (17) zu drücken.

3. Vorrichtung zur Behandlung der Wirbelsäule nach einem der Ansprüche 1 oder 2, **dadurch gekennzeichnet, daß** die Spannbohrung (20) schräg zur Achse (I) der ersten Bohrung (16) im Sinne einer Annährung zur Achse (I) der ersten Bohrung (16) beim Einschrauben der Klemmschraube (21) verläuft.

4. Vorrichtung zur Behandlung der Wirbelsäule nach einem der Ansprüche 1 bis 3, **dadurch gekennzeichnet, daß** die Druckfläche (22c) einen unteren, ebenen Teil hat, der allgemein in Richtung zum Boden der Querrinne (17) ausgerichtet ist, um zur Anlage an der Stabilisierungsstange (3) zu kommen, sowie einen oberen, nach oben offenen Teil, um den seitlichen Einsatz der Stabilisierungsstange (3) zu erleichtern.

5. Vorrichtung zur Behandlung der Wirbelsäule nach einem der Ansprüche 1 bis 4, **dadurch gekennzeichnet, daß** der Einsatz (22) frei um die Klemmschraube (21) drehbar ist und auf dem Gewindeschaft (21a) der Klemmschraube (21) axial durch einen Kragen (21c) der Klemmschraube (21) gehalten wird, der in eine ovale, innere Ringnut (22a) der Bohrung des Einsatzes (22) eingreift.

6. Vorrichtung zur Behandlung der Wirbelsäule nach einem der Ansprüche 1 bis 5, **dadurch gekennzeichnet, daß** der Endanschlagkragen (7) eine vorspringende, kugelförmige Abstützfläche (12) aufweist, die in Eingriff mit einer entsprechenden, hohlkugelförmigen Gegenfläche (13) am Eingang der ersten Bohrung (16) des Verbindungsschiebers (4) kommt, wobei ferner die erste Bohrung (16) des Verbindungsschiebers (4) einen Durchmesser hat, der größer als der Durchmesser des zylindrischen Gewindeabschnittes (5) des Verankerungselementes ist, um eine Winkelbewegung des Verbindungsschiebers (4) auf dem Verankerungselement vor dem Festschrauben der Klemmmutter (8) von der Achse (I) weg zu ermöglichen.

7. Vorrichtung zur Behandlung der Wirbelsäule nach einem der Ansprüche 1 bis 6, **dadurch gekennzeichnet, daß** die Stabilisierungsstange (3) in der Querrinne (17) in einer Zwischenstellung zwischen der Klemmschraube (21) und dem Verankerungselement (1, 2) gehalten ist.

8. Vorrichtung nach einem der Ansprüche 1 bis 7, **dadurch gekennzeichnet, daß** diese als Verankerungselement eine oder mehrere Knochenschrauben (1) mit zweifachem Gewinde hat, wobei der zylindrische Gewindeabschnitt (5) des Verankerungselementes das erste Gewinde und ein zweiter, koaxialer Gewindeabschnitt (9) gegenüber dem Endanschlagkragen (7) das zweite Gewinde bildet, das in den Knochen einschraubbar ist.

9. Vorrichtung nach einem der Ansprüche 1 bis 8, **dadurch gekennzeichnet, daß** diese als Verankerungselement einen oder mehrere Haken (2) aufweist, bei denen die Rückwand der Haken, die rechtwinklig zu der Achse des zylindrischen Gewindeabschnittes (5) verläuft, als Endanschlagkragen dient.

## Claims

1. Device for treatment of the spine, comprising :
- anchor members (1, 2) adapted to be anchored in vertebrae, each having a cylindrical screwthreaded portion (5) onto which is screwed a clamping nut (8), and an anchor portion connected to the cylindrical screwthreaded portion (5) by an intermediate stop flange (7),
- at least one circular section securing rod (3) having a smooth exterior surface,
- sliding connecting members (4) for connecting the anchor members (1, 2) to the securing rod (3), the sliding connecting members (4) including a first hole (16) conformed to have the cylindrical screwthreaded portion (5) of an anchor member passed through it to fasten it to an anchor member, the sliding connecting members having a top transverse groove (17) conformed to receive a section of the securing rod (3), with clamping means for selectively clamping and unclamping the securing rod (3) in said transverse groove (17),
**characterized in that** the clamping means comprise :
- a clamping screw (21), having a screwthreaded shank and a head,
- a clamping hole (20) in the bottom of the transverse groove of the sliding member, separate from the first hole (16) and offset laterally relative to the first hole (16),
- a cylindrical bearing surface (18) constituting a first edge of the transverse groove (17) and conformed to receive a section of the securing rod (3),
- an oblique bearing surface (19) constituting an opposite edge of the transverse groove (17) and inclined relative to the axis of the clamping hole,
- a clamping member (22) in the corner of the transverse groove (17) between the oblique bearing surface (19) and the securing rod (3) and adapted to be pushed toward the bottom of the transverse groove (17) by the clamping screw (21), having a bearing face (22b) bearing and sliding on the oblique bearing surface (19), and having an opposite thrust face (22c) bearing on the securing rod (3) to immobilize it in the transverse groove (17).

2. Device according to claim 1 for treatment of the spine, **characterized in that** the clamping member (22) has a hole through it through which the clamping screw (21) passes, so that the head (21b) of the clamping screw (21) bears on the external face of the clamping member (22) to push it toward the bottom of the transverse groove (17).

3. Device according to claim 1 or claim 2 for treatment of the spine, **characterized in that** the clamping hole (20) is oriented obliquely to the axis (I) of the first hole (16), in the direction approaching the axis (I) of the first hole (16) on screwing of the clamping screw (21).

4. Device according to any one of claims 1 to 3 for treatment of the spine, **characterized in that** the thrust face (22c) has a plane lower portion oriented generally toward the bottom of the transverse groove (17) to bear against the securing rod (3), and an upper portion open at the top to facilitate lateral engagement of the securing rod (3).

5. Device according to any one of claims 1 to 4 for treatment of the spine, **characterized in that** the clamping member (22) turns freely about the clamping screw (21), and is retained axially on the screwthreaded shank (21a) of the clamping screw (21) by a flange (21c) of the clamping screw (21) engaged in an oval annular groove (22a) inside the hole in the clamping member (22).

6. Device according to any one of claims 1 to 5 for treatment of the spine, **characterized in that** the stop flange (7) has a male spherical bearing surface (12) engaging in a corresponding female spherical surface (13) at the entry of the first hole (16) in the sliding connecting member (4), the first hole (16) in the sliding connecting member (4) having a diameter greater than the diameter of the cylindrical screwthreaded portion (5) of the anchor member to allow angular oscillation of the sliding connecting member (4) on the anchor member and away from the axis (I) before screwing of the clamping nut (8).

7. Device according to any one of claims 1 to 6 for treatment of the spine, **characterized in that** the securing rod (3) is engaged, in the transverse groove (17), in an intermediate position between the clamping screw (21) and the anchor member (1, 2).

8. Device according to any one of claims 1 to 7 for treatment of the spine, **characterized in that** it comprises anchor members in the form of one or more twin-thread bone screws (1), the cylindrical screwthreaded portion (5) of an anchor member constituting the first screwthread, and a coaxial second screwthreaded portion (9), on the opposite side of the stop flange (7), constituting a second screwthread adapted to screw into bone.

9. Device according to any one of claims 1 to 8 for treatment of the spine, **characterized in that** it comprises anchor members in the form of one or more hooks (2) where the rear wall of hooks, perpendicular to the axis of the cylindrical screwthreaded portion (5), serves as a stop flange.
